# EUROPEAN PATENT SPECIFICATION

(11) **EP 2 070 532 B1**
(45) Date of publication and mention of the grant of the patent: **24.07.2013**
(21) Application number: 07829030.1
(22) Date of filing: 02.10.2007
(51) Int. Cl.: A61K 31/36, A23C 15/00, A23D 7/00, A23L 1/24, A61K 9/113, A61K 31/34, A23D 7/005, A61K 9/48, A61K 9/00, A23L 1/308, A23K 1/16

(54) **O/W/O EMULSION CONTAINING LIGNAN COMPOUNDS, AND COMPOSITION CONTAINING THE SAME**
Ö/W/Ö EMULSION MIT EINER LIGNAN-VERBINDUNG UND ZUSAMMENSETZUNG DAMIT
ÉMULSION D' HUILE/EAU/HUILE CONTENANT UN COMPOSÉ DE LIGNANE, ET COMPOSITION LA CONTENANT

(30) Priority: 04.10.2006 JP 2006272781
(43) Date of publication of application: 17.06.2009
(73) Proprietor: Suntory Holdings Limited, Kita-ku, Osaka-shi Osaka 530-8203 (JP)
(72) Inventor: NISHIUMI, Toshihiro, Mishima-gun, Osaka 6180001 (JP); ONO, Yoshiko, Mishima-gun, Osaka 6188503 (JP); TOMIMORI, Namino, Shimamoto-cho, Mishima-gun, Osaka 618-8503 (JP); NAKAHARA, Koichi, Kawasaki-shi, Kanagawa 211-0067 (JP)
(74) Representative: Vossius & Partner
(86) International application number: PCT/JP2007/069290
(87) International publication number: WO 2008/044550

(56) References cited:
- AU-B2- 2005 320 579
- JP-A- 02 200 145
- JP-A- 63 044 842
- JP-A- 63 044 843
- US-A- 5 438 041
- US-A1- 2007 166 255

## Description

### TECHNICAL FIELD

The present invention relates to O/W/O emulsions containing lignan-class compounds and compositions containing the same; more particularly, the present invention relates to compositions that are improved in the amount of bodily absorption of lignan-class compounds.

### BACKGROUND ART

Lignan-class compounds have been reported to have a variety of in vivo actions. For example, USP 4427694 discloses the effectiveness of sesamin in alleviating the symptoms of alcohol intoxication and/or alcohol or tobacco withdrawal, and JP 2-138120 A discloses the effectiveness of sesaminol and episesaminol in the treatment and prevention of allergosis such as bronchial asthma. The assignees of the subject application also confirmed various physiological actions of lignan-class compounds and, to date, they have revealed such effects as the blood cholesterol lowering action (Japanese Patent No. 3001589), the action of inhibiting Δ⁵-unsauration enzymes (Japanese Patent No. 3070611), the action of improving hepatic functions (Japanese Patent No. 3075358), cholesterol depression (Japanese Patent No. 3075360), the action of preventing sickness from drinking (Japanese Patent No. 3124062), the action of inhibiting the metabolism of cholesterol and bile acid, as well as lowering cholesterol (Japanese Patent No. 3283274), the carcinogenesis suppressing action (Japanese Patent No. 3183664), the breast cancer suppressing action (JP 05-043458 A), as well as the action of suppressing the generation of lipid peroxides (JP 05-051388 A), and the action of scavenging active oxygen (JP 06-227977 A).

However, lignan-class compounds are hardly soluble in water and, what is more, they dissolve to only some extent in organic solvents that can be used in medicaments or foods. Such sparingly soluble substances have the problem of not being easily absorbed in the living body.

As a method of improving the bodily absorption of fat-soluble substances, it has been proposed to make finer micelles of fat-soluble substances (render them in finer particles). This exploits such a nature of fat-soluble substances that the smaller the size of their particles, the more advantageous they are in terms of absorption by the digestive tract. To give a specific example, JP 2004-196781 A discloses a coenzyme Q10 containing water-soluble composition that comprises coenzyme Q10, a specified polyglycerin, fatty acid monoester, etc. and which is markedly improved in bodily absorption by adjusting the average particle size to 110 nm or smaller. As another example, JP 9-157159 A discloses a carotinoids containing composition comprising an oil phase that has carotinoids dissolved in oil or fat and that is emulsified in a water phase containing a polyglycerin fatty acid ester, lecithin and a polyhydric alcohol and which has the bodily absorption of a sparingly soluble substance, carotenoid, improved by adjusting the average particle size of the oil phase to 100 nm or smaller.

On the other hand, a means called double emulsification (O/W/O; oil-in-water-in-oil emulsion) is sometimes adopted for the purpose of improving the flavor and palatability or suppressing the deterioration of fat-soluble substances. Specifically, JP 5-130843 A, for example, discloses an oil-in-water-in-oil emulsified oil or fat composition that has egg yolk and a saccharide incorporated in a water phase as a product that can solve the problem (oiliness) with the conventional O/W emulsion having only egg yolk incorporated. In addition, JP 7-313055 A shows that if DNA susceptible to oxidative deterioration is incorporated in the internal oil phase of an O/W/O emulsion, the DNA is covered with the film of the emulsified phase to ensure that it is sufficiently protected against deterioration in quality to provide a product having good flavor and palatability. Further in addition, JP 2004-97113 A shows that by forming an O/W/O emulsion of fat-soluble vitamins, the activity of the vitamins can be maintained to survive storage for a prolonged time.
[Patent Document 1] USP 4427694
[Patent Document 2] JP 2-138120 A
[Patent Document 3] Japanese Patent No. 3001589
[Patent Document 4] Japanese Patent No. 3070611
[Patent Document 5] Japanese Patent No. 3075358
[Patent Document 6] Japanese Patent No. 3075360
[Patent Document 7] Japanese Patent No. 3124062
[Patent Document 8] Japanese Patent No. 3283274
[Patent Document 9] Japanese Patent No. 3183664 (JP 04-159221 A)
[Patent Document 10] JP 05-043458 A
[Patent Document 11] JP 05-051388 A
[Patent Document 12] JP 06-227977 A
[Patent Document 13] JP 2004-196781 A
[Patent Document 14] JP 9-157159 A
[Patent Document 15] JP 5-130843 A
[Patent Document 16] JP 7-313055 A
[Patent Document 17] JP 2004-97113 A

### DISCLOSURE OF THE INVENTION

### PROBLEMS TO BE SOLVED BY THE INVENTION

The present inventors have conducted studies with a view to enhancing the bodily absorption of lignan-class compounds. And they first found that when an oil-in-water emulsion prepared by emulsifying in a water phase an oil phase having a lignan-class compound dissolved therein was administered perorally, the time to maximum blood level (Tmax) was markedly shortened compared to the case where the lignan-class compound was simply dissolved in oil or fat (PCT/JP 2006/306845). This improvement in the rate of bodily absorption is particularly useful in the case where the effect of the lignan-class compound is desirably exhibited soon after it is ingested, for example, in the case where the effectiveness in preventing sickness from drinking or the effectiveness in scavenging active oxygen is wanted.

On the other hand, the investigation by the present inventors has revealed that the above-described means excels in its ability to improve the rate of bodily absorption but does not improve the amount of bodily absorption. To state specifically, whether a lignan-class compound was administered perorally as an oil-in-water emulsion or as a simple solution in fat or oil, the area under the blood concentration-time curve (AUC) was the same (see [Reference Example] herein).

Therefore, the present invention particularly focuses on the amount of bodily absorption of lignan-class compounds and aims to make it greater than it has been in the prior art.

### MEANS FOR SOLVING THE PROBLEMS

The present inventors conducted intensive studies in order to enhance the amount of bodily absorption of lignan-class compounds; as a result, it was surprisingly found that when a lignan-class compound as dissolved in the internal oil phase of an O/W/O emulsion was administered perorally, the amount of its bodily absorption could be improved over the prior art. Conventionally, double emulsification has been performed for the purpose of improving flavor or palatability or suppressing the deterioration of fat-soluble substances and it has not been known at all that it is capable of improving the bodily absorption of fat-soluble substances.

In short, the present invention provides a double emulsified lignan-class compound, more particularly, an O/W/O emulsion composition having at least one of lignan-class compounds dissolved in an internal oil phase, as well as a method of improving the amount of bodily absorption of lignan-class compounds by means of such a composition.

The present invention also provides an O/W/O emulsion composition which, when administered perorally, enables at least one of lignan-class compounds to be absorbed with a greater AUC (preferably with an AUC at least 1.13 times, more preferably at least 1.25 times, even more preferably at least 1.5 times, and most preferably at least 1.6 times) than when the same quantity of that lignan-class compound is dissolved in the same oil or fat as the internal oil phase and administered perorally under the same conditions; the present invention further provides a process for producing such O/W/O emulsion composition.

A test for checking to see if a certain O/W/O emulsion composition (test preparation) has a greater AUC than a reference preparation can be designed as appropriate by any skilled artisan through adjustment of the test and like factors.

### BRIEF DESCRIPTION OF THE DRAWINGS

[FIG. 1] FIG. 1 is a graph showing the time course of the total sum of sesamin and episesamin levels (sesamin + episesamin level) in the blood of rats administered with the composition of the present invention or a comparative composition.
[FIG. 2] FIG. 2 is a graph showing the amount of bodily absorption (AUC) in rats administered with the composition of the present invention or the comparative composition.
[FIG. 3] FIG. 3 is a graph showing the time course of the total sum of sesamin and episesamin levels (sesamin + episesamin level) in the blood of rats administered with the emulsion of the present invention (average particle size ≒ 100 nm) or a comparative composition.

### BEST MODE FOR CARRYING OUT THE INVENTION

The composition of the present invention can be produced by a process comprising the following steps:
1) dissolving at least one of lignan-class compounds in oil or fat to prepare a lignan-class compound dissolving liquid;
2) emulsifying the lignan-class compound dissolving liquid in a water phase to form an O/W emulsion; and
3) further emulsifying the O/W emulsion in an oil phase to prepare an O/W/O emulsion.

### Lignan-class compounds

The lignan-class compounds to be used in the present invention include sesamin, sesaminol, episesamin, episesaminol, sesamolin, 2-(3,4-methylenedioxyphenyl)-6-(3-methoxy-4-hydroxyphenyl)-3,7-dioxabicyclo[3,3,0]octane, 2,6-bis-(3-methoxy-4-hydroxyphenyl)-3,7-dioxabicyclo[3,3,0]octane, and 2-(3,4-methylenedioxyphenyl)-6-(3-methoxy-4-hydroxyphenoxy)-3,7-dioxabicyclo[3,3,0]octane; these compounds may be used either alone or in admixture.

The above-mentioned lignan-class compounds are in no way limited with respect to their form, the process for their production, and so forth. For example, one may use the extract from sesame oil as obtained by a known method (such as the method comprising adding hot methanol to the sesame oil for extraction, removing the methanol from the extract, then adding acetone to the residue for extraction (this method is described in JP 4-9331 A)) (the extract containing a high proportion of lignan-class compounds or being optionally purified); if desired, commercial sesame oil (in liquid form) can also be used. However, if sesame oil is used, its characteristic flavor may sometimes be evaluated as being unfavorable from an organoleptic viewpoint, so it is preferred to use the tasteless and odorless extract from sesame oil that contains a high proportion of lignan-class compounds or the purified product of such extract. Another problem with the use of sesame oil is that the content of lignan-class compounds is so low that if one attempts to incorporate a preferred amount of lignan-class compounds, the composition to be formulated that contains the lignan-class compound containing oil-in-water emulsion needs to be ingested in an excessive amount that might cause some inconvenience in ingestion. Therefore, from the additional viewpoint of the need to ingest only a small amount of the composition, it is preferred to use the extract from sesame oil that contains a high proportion of lignan-class compounds or the pure form of lignan-class compounds that have been isolated and purified. It should be noted here that the extract from sesame seeds and the like that contain a high proportion of lignan-class compounds has the good smell of sesame, so if it is used in the food or beverage for animals according to the present invention, the aroma of sesame can be imparted to it.

Lignan-class compounds can also be obtained by synthesis. Exemplary methods include the method of Beroza et al. for sesamin and episesamin (J. Am. Chem. Soc., 78, 1242 (1956)), as well as the method of Freundenberg et al. for pinoresinol (Chem. Ber., 86, 1157 (1953)) and the method of Freundenberg et al. for siringaresinol (Chem. Ber., 88, 16 (1955)).

Further, the lignan-class compounds can be used in the form of glycosides and, in addition, these can be used either alone or in suitable combinations as components of the composition.

### O/W/O type emulsion composition

According to the present invention, there is provided an O/W/O type emulsion composition containing a lignan-class compound. The lignan-class compound is dissolved in at least the internal oil phase. The lignan-class compound can be dissolved in both the internal oil phase and the external oil phase.

The term "internal oil phase" as used herein means, except in a special case, the oil phase located in the innermost part of the O/W/O emulsion composition. The internal oil phase is sometimes referred to as the "innermost oil phase." Specific examples of the "inner oil phase" in the present invention include not only sesame oil and a sesame oil extract containing a high proportion of lignan-class compounds that remain dissolved in sesame oil (sesame oil concentrate) but also a sesame extract, as well as refined lignan-class compounds and other powdered forms (solid forms) of lignan-class compounds that are dissolved in fat or oil.

The fat or oil in which lignan-class compounds are to be dissolved is not limited in any particular way and those which can be added to foods or pharmaceuticals and can dissolve lignan-class compounds may be used either alone or in admixture of two or more species. Specific examples include: natural oils and fats such as almond oil, safflower oil, apricot kernel oil, avocado oil, evening primrose oil, wheat germ oil, corn oil, sunflower oil, safflower oil, walnut oil, olive oil, castor oil, kukui nut oil, grape seed oil, cocoa butter, coconut oil, soybean oil, rapeseed oil, peanut oil, rice oil, sesame oil, palm kernel oil, palm oil, jojoba oil, macadamia nut oil, shea butter, mango butter, kokum butter, whale oil, sardine oil, and squid oil; and synthetic oils or fats such as margarine; while fat or oil that contain as a main ingredient the diacylglycerol contained in the above-mentioned olive oil and the like, as well as fat or oil that contain as a main ingredient the middle-chain fatty acid triglyceride (MCT) contained in palm kernel oil and the like can also be used, those oils or fats which contain large amounts of saturated fatty acids are particularly preferred since they are not readily oxidized. In addition, not only fats or oils that are liquid at ordinary temperatures but also those which are mixed with semi-solid or solid lard, tallow, hydrogenated fish oil, margarine, shortening, and the like may be used. When the lignan-class compounds or the extract that contains a high proportion of lignan-class compounds is to be dissolved in fats or oils, they may, depending on the need, be heated for dissolution or otherwise treated.

The "water phase" as used herein means, except in a special case, the water phase of the O/W/O emulsion composition. The "water phase" is not limited in any particular way as long as it is an aqueous medium; examples include not only water and aqueous solutions but also a variety of aqueous drinks such as common drinks like juice drinks, carbonated drinks, cow's milk, soymilk, cereal drinks, coffee, green tea, etc., and alcoholic beverages.

If desired, a solubilizing agent may be added to the water phase for the purpose of increasing the percent content of the internal oil phase. Examples of such solubilizing agent include propylene glycol, ethanol, mono- or disaccharides, and sugar alcohols (e.g. sorbitol, xylitol, and mannitol).

The term "oil phase" as used herein means, except in a special case, the oil phase located in the outermost part of the O/W/O emulsion composition. The oil phase may generally sometimes be referred to as the "outermost oil phase." As the "oil phase" in the present invention, any type can be used without limitation as long as it is oil or fat that can be added to food or medicines and which can emulsify the water phase in which the internal oil phase has been emulsified (O/W emulsion); a single type of oil phase can be used alone or a plurality of types can be used in admixture. Specifically, the various types of oil and fat that have been described above in the explanation of the "internal oil phase" may be used.

To produce the lignan-class compound containing O/W/O emulsion composition of the present invention, any known methods may be used as long as the lignan-class compound is dissolved in the internal oil phase, but it is preferred to adopt the two-stage emulsification method. Into the oil or fat that serves as the oil phase, an internal phase O/W emulsion that has been preliminarily prepared in the usual manner may be re-emulsified to obtain the O/W/O type emulsion composition. Hereinafter, the process for producing the O/W/O type emulsion composition by this two-stage emulsification method is described.

### [First-stage emulsification treatment: O/W emulsion]

To prepare the lignan-class compound containing O/W/O emulsion composition of the present invention, the first-stage emulsification treatment is conducted to prepare an O/W emulsion.

The first-stage emulsification treatment starts with preparing a liquid in which a lignan-class compound is dissolved (the internal oil phase). As already mentioned, a liquid having sesame oil or the like dissolved therein may be used as such or, alternatively, a powdered form of lignan-class compound may be added to the solvent oil or fat and mixed, preferably agitated with heating so that it is fully dissolved. The blending ratio between the lignan-class compound and the fat or oil varies with the type of the lignan-class compound and the solvent fat or oil and it can be set appropriately in consideration of this fact; generally, lignan-class compounds are fully dissolved when the blending ratio (by weight) between lignan-class compound and oil or fat is such that the lignan-class compound to solvent ratio is about 1:15-2000, preferably about 1:15 to 100.

By mixing this internal oil phase with the water phase and homogenizing the mixture to emulsify it, there is obtained an O/W type emulsion having the oil droplets dispersed in the water.

The mixing ratio (by weight) between the internal oil phase and the water phase can be set as appropriate; for example, the ratio of the internal oil phase to the water phase can be set at 100:2-200, or alternatively, at 100:5-50.

The physical techniques for achieving homogenization are not limited in any way and may be exemplified by the membrane emulsification method suing a porous membrane, and the agitation method using such an apparatus as an agitating emulsifier, a high-pressure homogenizer, an ultrasonic emulsifier, an ultra-mixer, or a colloid mill. According to the review by the present inventors, if no homogeneous emulsion is formed, namely, if the dispersion stability of oil droplets in emulsion is poor, AUC may sometimes decrease. In order to obtain a homogeneous O/W emulsion, a surfactant may advantageously be added to the water phase and/or the internal oil phase. Surfactants may be selected as appropriate for the types and amounts of lignan-class compounds, as well as oils and fats; examples include glycerin fatty acid esters, sucrose fatty acid esters, sucrose acetate isobutyrate, sorbitan fatty acid esters, propylene glycol fatty acid esters, calcium stearyl lactate, soybean saponin, lecithin, wheat protein digest, gelatin, carboxymethylcellulose, carboxymethylcellulose sodium, gum arabic, xanthan gum, arabinogalactan, dextrin, casein, and casein sodium; these surfactants may be used either alone or in admixture. The present inventors have confirmed that the O/W/O emulsion composition can be obtained by using glycerin fatty acid esters or enzyme-decomposed soybean lecithin and that, in particular, a homogeneous O/W/O emulsion composition can be obtained when decaglycerin monolauric acid ester is used. [See Example 1.]
The average particle size of the oil droplets of the internal oil phase is not limited in any particular way as long as it is effective in ensuring that the composition, when administered perorally, enables at least one of lignan-class compounds to be absorbed with a greater AUC than when the same quantity of that lignan-class compound is dissolved in the same oil or fat as the internal oil phase and administered perorally under the same conditions; according to the investigation conducted by the present inventors to study the absorption rate, satisfactory absorption was observed in each of the emulsions having average particle sizes of 100 nm, 130 nm, and 250 nm. When preparing the O/W/O emulsion by the two-stage emulsification method, it is important that the first emulsified system be rendered sufficiently stable. To this end, it is recommended that the oil droplets of the internal oil phase in the first emulsified system have average particle sizes of not more than 1000 nm. Furthermore, it is preferred that the oil droplets of the internal oil phase are so prepared that their average particle size is smaller than the pore size of the porous membrane that is used in the step of re-emulsification. In addition, it is generally known that with the decreasing particle size, the surface area increases, thus contributing to increased electrostatic stability and improved dispersion stability. Therefore, in order to obtain a homogeneous emulsion, it is also effective to reduce the particle size of oil droplets that compose the dispersion phase (i.e., render them finer). Specifically, the average particle size of oil droplets may be adjusted to 1000 nm or less, preferably 500 nm or less, more preferably 300 nm or less. At 300 nm or less, the emulsion can be left to stand at room temperature for 2 days without causing segregation of the oil phase, thus showing satisfactory dispersion stability.

The first-stage O/W emulsion can be produced by mixing the internal oil phase with the water phase and homogenizing the mixture; in order to produce an emulsion containing the above-described fine oil droplets with an average particle size of 1000 nm or less, preferably 500 nm or less, more preferably 300 nm or less, a porous membrane having a uniform pore size may be employed to perform the membrane emulsification method or, alternatively, preliminary emulsification that consists of mixing the oil and water phases may be followed by a means of further emulsification (main emulsification) until the average particle size of the oil droplets comes to be within the above-mentioned ranges.

The membrane emulsification method is one in which a porous membrane is placed between a liquid (a) that provides a disperse phase (internal oil phase) and a liquid (b) that provides a continuous phase (water phase) and the liquid (a) is forced into the liquid (b) through the membrane by such means as nitrogen gas. The porous membrane may be inorganic or organic as long as it has a uniform pore size (the term "uniform pore size" as used herein assumes the case where the pore size of the porous membrane is such that the pore size (φ10) at which the pore volume accounts for 10% of the total volume, as divided by the pore size (φ90) at which the pore volume accounts for 90% of the total volume, on the relative cumulative pore distribution curve is within the range of from about 1 to about 1.5); examples that can be used include the CaO-B₂O₃-SiO₂-Al₂O₃ based porous glass that is disclosed in JP 62-25618 B, the CaO-B₂O₃-SiO₂-Al₂O₃-Na₂O based porous glass and CaO-B₂O₃-SiO₂-Al₂O₃-Na₂O-MgO based porous glass that are disclosed in JP 61-40841 A (USP 4,657,875), and the microporous glass (SPG membrane; CaO-Al₂O₃-B₂O₃-SiO₂ based porous glass membrane) that is disclosed in JP 2002-302414 A. The membrane emulsification method, which is known to permit the size of particles to be designed in accordance with the specific use, is a production process that can advantageously be used in the present invention. It should also be noted that if a glass porous membrane in cylindrical form is used as the porous membrane, the O/W emulsion can also be prepared by such a method that with the liquid (b) being circulated within the cylinder, the liquid (a) is forced in from the outside by such means as nitrogen gas.

The average size of the pores can be chosen as appropriate for the average size of the particles in the O/W emulsion to be obtained and it is usually about 0.1-10 µm, preferably about 0.1-5 µm, and more preferably about 0.1-0.3 µm. Another consideration that may be taken is that the size of the particles in the emulsion to be formed is generally equal to the pore size of the membrane times 3.25.

The thickness of the porous membrane is not limited in any particular way but it is usually about 0.1-1.5 mm. In the membrane emulsification method, the pressure required to force in the liquid (a) can be set as appropriate for the type of the disperse phase (internal oil phase), the type of the continuous phase (water phase), the type and concentration of the surfactant, etc. and it is usually about 20 Ka to about 5 MPa.

Membrane emulsification is preferably performed under heating. For example, in the present invention, an SPG membrane with pore sizes of about 0.1-0.2 µm may be provided and the water-phase component is allowed to flow on one of its sides as it is heated; at the same time, the lignan-class compound containing oil-phase component (internal oil phase) is heated at about 80-90 °C and the heated oil-phase component is placed under pressure so that it is forced into the SPG membrane heated at about 80-90 °C, whereby an O/W (oil-in-water) emulsion having particle sizes of about 0.3-0.8 µm can be obtained. The thus obtained lignan-class compound containing emulsion will not experience any change with time, such as crystallization of the emulsion particles or the particles joining together to have their size varied; in other words, the emulsion has extremely good stability over time.

In the agitation method, the apparatus that can be employed is not limited in any particular way as long as it is capable of high-speed agitation and specific examples include those which were already mentioned for the homogenizing treatment and are exemplified by such apparatuses as an agitating emulsifier, a high-pressure homogenizer, an ultrasonic emulsifier, an ultra-mixer, and a colloid mill. Agitation conditions may be set as appropriate for the type and shape of the apparatus used, as well as the properties and quantity of the object to be agitated (the mixture of oil and water phases) and they are typically about 10-30 minutes at 5000-30000 rpm, preferably at 6000-20000 rpm.

In the method involving the high-pressure homogenizer, homogenizers capable of homogenization at, for example, 9.8 MPa (100 kgf/cm²) and above [including, for example, MICROFLUIDIZER (trade name of MIZUHO INDUSTRIAL CO., LTD.) and GORIN HOMOGENIZER (trade name)] may be employed.
The conditions for preparing the O/W emulsion using these apparatuses may be selected as appropriate and it is desirably prepared at a pressure of, say, about 9.8-245 MPa (100-2500 kgf/cm²), preferably about 49-196 MPa (500-2000 kgf/cm²), at room temperature or under optional heating.

### [Second-stage emulsification treatment: O/W/O type emulsion composition]

To prepare the lignan-class compound containing O/W/O emulsion composition of the present invention, the first-stage emulsification treatment is followed by the second-stage emulsification treatment to prepare an O/W/O emulsion.

In the second-stage emulsification treatment, the O/W emulsion obtained in the first stage is mixed with the oil phase and then emulsified by homogenizing the mixture, whereupon there is obtained an O/W/O emulsion composition having the droplets of the O/W emulsion dispersed in the oil.

The mixing ratio (by weight) between the O/W emulsion and the oil phase can be appropriately set; for example, the ratio of the O/W type emulsion to the oil phase can be set at 100:10-1000, or alternatively, at 100:25-500.

The physical techniques for achieving homogenization are not limited in any way and may be exemplified by the agitation method using such an apparatus as an agitating emulsifier, a high-pressure homogenizer, an ultrasonic emulsifier, an ultra-mixer, or a colloid mill; alternatively, a liquid (c) that serves as a continuous phase (external oil phase) may be provided and the O/W emulsion is be emulsified in that phase by the membrane emulsification method, whereby the O/W/O emulsion can be prepared. It should be noted here that the liquid (c) serving as the external oil phase may be oil or fat, to which a lignan-class compound or any other additive may be added. In the membrane emulsification method, the preliminarily prepared O/W emulsion is forced into the liquid (c) through the above-described type of porous membrane, whereupon there is obtained an O/W/O emulsion characterized by a uniform size of emulsion particles. In this case, the above-described type of porous membrane having a uniform pore size may be used; if a glass porous membrane is to be used as such membrane, the following precaution should be taken: the glass porous membrane is inherently hydrophilic, so in the case of preparing the O/W/O emulsion, it is usually subjected to a variety of surface treatments so that its surface is rendered hydrophobic before use.

The average size of the pores can be chosen as appropriate for the average size of the particles in the O/W/O emulsion to be obtained and it is usually about 0.15-30 µm, preferably about 0.3-5 µm, and more preferably about 0.5-3 µm. Another consideration that may be taken is that the size of the particles in the emulsion to be formed is generally equal to the pore size of the membrane times 3.25.

### [Other additives and the like]

Aside from the above-mentioned lignan-class compound, oil or fat, water-based solvent, and the surfactant, the O/W/O emulsion composition of the present invention may contain mixed therein vitamin C, vitamin E, d-α-tocopherol, ellagic acid, erythorbic acid, sodium erythorbate, ethylenediaminetetraacetic acid disodium salt, dibutyl hydroxytoluene, sodium L-ascorbate, pherol and the like as antioxidants for the purpose of preventing oxidation. If necessary, a sweetener, a seasoning, a sour agent, a pH modifier and the like may be added.

With the O/W/O emulsion composition of the present invention, use of a high-melting oil or fat in the oil phase contributes to yielding a composition of good shape retention whereas use of a high-melting oil in the internal oil phase contributes to affording a composition characterized by suppressed taste, odor, etc. of the internal oil phase.

### Uses

The present invention contributes to improving the absorbability of lignan-class compounds in the living body. Hence, the O/W/O emulsion composition of the present invention can be used in the form of various food compositions or oral pharmaceutical compositions which can benefit from the improvement in the amount of absorption of lignan-class compounds. The food compositions of the present invention also include those in the form of drinks. The food compositions of the present invention can be formulated as food with nutrient function claims, food for specified health use, health food, nutritional supplement, health drink, soft capsule, etc.

The ratio (by weight) at which the O/W/O emulsion composition of the present invention is blended in the food composition or oral pharmaceutical composition can be appropriately set for the purpose of incorporating the lignan-class compound at a desired concentration in a desired amount and it may range from about 1 to 100 wt%. In addition, the food composition or oral pharmaceutical composition of the present invention may use a variety of acceptable additives, such as excipient, binder, disintegrant, lubricant, coating agent, suspending agent, emulsifier, stabilizer, preservative, and buffer.

In the pharmaceutical composition of the present invention, the amount of the lignan-class compound as the active ingredient, the duration of its administration, and the interval between administrations can be set as appropriate for the specific object, symptom, the age and body weight of the subject to be treated, and other factors.

The subject to which the food composition or oral pharmaceutical composition of the present invention may be applied is humans or animals. The term "animals" refers to industrial animals, pets, and laboratory animals; specifically, the term "industrial animals" refers to animals that need be bred for industrial purposes and they include farm animals such as cattle, horse, swine, goat, sheep, etc., poultry such as chicken, duck, quail, turkey, ostrich, etc., and fishes such as adult yellowtail, young yellowtail, red sea bream, common horse mackerel, carp, rainbow trout, eel, etc; the term "pets" refers to so-called pet animals or companion animals such as dog, cat, marmoset, little bird, hamster, goldfish, etc.; the term "laboratory animals" refers to rat, guinea pig, beagle, miniature pig, rhesus monkey, crab-eating monkey, and other animals that are subjected to research in such fields as medicine, biology, agronomy, pharmacy, etc.

### Method of evaluation

If lignan-class compounds are administered orally according to the present invention, AUC is improved as compared to the case where they are simply dissolved in fat or oil and administered under the same conditions. Such an improvement in absorbability into the body can be evaluated by measuring the level of lignan-class compounds in blood over time.

The level of lignan-class compounds in blood can be determined by the following procedure: blood is collected and subjected to a centrifugal operation to obtain a plasma sample, to which is added an internal standard (e.g., YUDESMIN produced by Funakoshi Corporation); thereafter, the solid phase is extracted with a solid-phase extracting polymer packing agent (e.g., Oasis HLB produced by Waters Corporation) and the liquid extract is concentrated under vacuum; the concentrate is then suspended in methanol, passed through a filter, and subjected to LC-MS/MS for quantification of the lignan-class compounds.

In the case where a plurality of lignan-class compounds are used, the total sum of their blood levels may be plotted against time and AUC determined for evaluation purposes. Simultaneously with AUC, Cmax and Tmax may also be determined.

It should be noted here that the term average particle size as used herein means, except in special cases, the median size (the particle size corresponding to 50% on a plus-mesh distribution curve; sometimes referred to as a 50% particle size) and this can be known by the method of light scattering, particle size distribution measurement. The method of dynamic, light scattering, particle size distribution measurement may also be adopted.

On the following pages, the present invention is described more specifically by showing working examples and comparative examples but it should be understood that the present invention is by no means limited to the following working examples.

### [EXAMPLE 1] Production of O/W/O Emulsion Composition

A 0.1362-g portion of sesamin (product of TAKEMOTO OIL & FAT Co., Ltd.; sesamin/episesamin = 51.1:48.2) was suspended in 100 mL of olive oil that had been heated to 80 °C and the suspension was agitated for 20 minutes until the sesamin dissolved uniformly. Subsequently, by the membrane emulsification method using a hydrophilic Shirasu Porous Glass (SPG) membrane (product of SPG TECHNOLOGY Co., Ltd.; pore size, 0.2 µm), 12 mL of the olive oil having the sesamin dissolved therein (disperse phase) was continuously emulsified in 20 mL of pure water having 1.0 wt% of a surfactant dissolved therein (continuous phase), whereupon a sesamin-containing water-soluble emulsified composition (O/W emulsion) was obtained. In this process, an extruding pressure of 0.6 MPa was applied. Further in addition, by the membrane emulsification method using a hydrophobic SPG membrane (product of SPG TECHNOLOGY Co., Ltd.; pore size, 1.3 µm), 15 mL of the resulting sesamin-containing water-soluble emulsified composition (disperse phase) was continuously emulsified in 15 mL of olive oil having 1.0 wt% of a condensed hexaglycerin ricinoleic acid ester (SUN SOFT No. 818SK; product of Taiyo Kagaku Co., Ltd.) dissolved therein (continuous phase), whereupon 30 mL of a sesamin-containing oil-soluble emulsified composition was obtained. In this process, an extruding pressure of 0.4 MPa was applied. Note that the SPG (*Shirasu* Porous Glass) used in this Example is a porous glass made from the volcanic ash *shirasu* and has a unique porous structure in which a lot of pores intertwine. It is characterized by a uniform pore size which yet can be controlled. The following four types of surfactant were used to emulsify the disperse phase: decaglycerin monolauric acid ester (SUN SOFT Q-12S), decaglycerin monomyristic acid ester (SUN SOFT Q-14S), pentaglycerin monooleic acid ester (SUN SOFT A-171E), and enzyme-decomposed soybean lecithin (SUN LECITHIN A-1), each being available from Taiyo Kagaku Co., Ltd.

The thus obtained sesamin-containing oil-soluble emulsified compositions were checked for their homogeneity under a microscope and, whichever surfactant was used, the O/W/O emulsion was confirmed to have been formed. Particularly in the case where the decaglycerin monolauric acid ester was used, there was obtained an O/W/O emulsion with small particle size and high uniformity.

### [EXAMPLE 2] Sesamin's Bodily Absorption Test on O/W/O Emulsion

Among the O/W/O emulsions produced in Example 1, the sesamin-containing O/W/O type emulsion produced by using the decaglycerin monolauric acid ester as a surfactant was subjected to a sesamin's bodily absorption test (test sample).

For comparison, 0.1 g of sesamin (product of TAKEMOTO OIL & FAT Co., Ltd.) was suspended in 99.9 g of olive oil that had been heated to 80 °C and the suspension was agitated for 20 minutes until the sesamin dissolved uniformly (comparative sample).

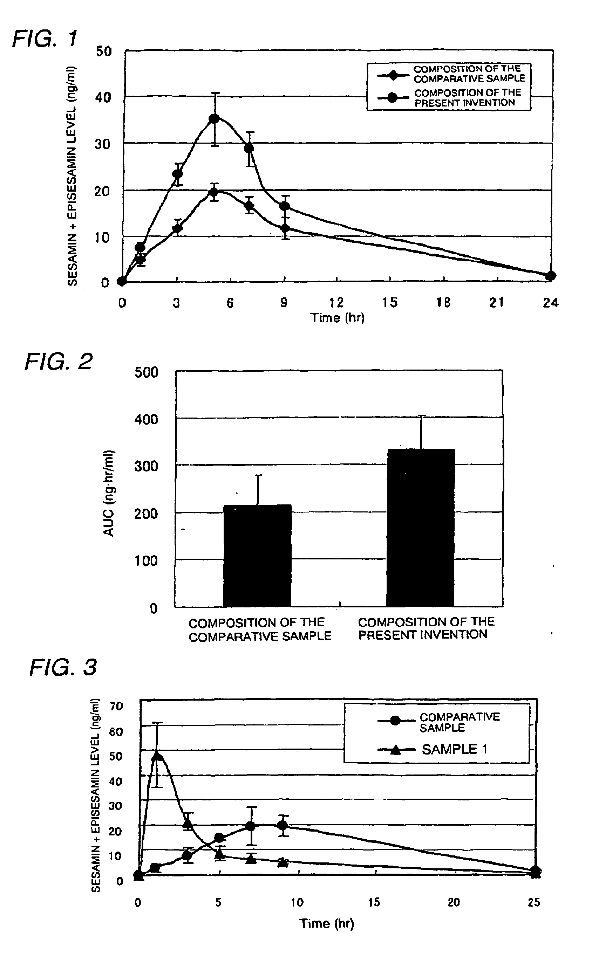

SD (IGS) male rats (9-week old) were purchased from CHARLES RIVER LABORATORIES, JAPAN, INC. and acclimatized in the test environment for a week; the animals that were shown to have grown normally were subjected to the test. The rats that were fasted overnight were divided into two groups, each consisting of 6 animals, and using a stomach tube, they were perorally administered with the finely ground lignan-class compound containing aqueous solution as the test sample or the olive-dissolved fat or oil of sesamin as the comparative sample at a dose of 9 mg/10 mL/kg. At 1, 3, 5, 7, 9 and 24 hours after the start of administration, blood was withdrawn from the tail vein of each animal, collected into a heparinized blood collecting tube, and centrifuged (8000 rpm, 10 min) to obtain plasma samples. After adding an internal standard, the solid phase was extracted with Oasis HLB and the liquid extract was concentrated under vacuum; the concentrate was suspended in methanol, passed through a filter and subjected to LC-MS/MS to quantitate sesamin and its isomer, episesamin. According to the usual method, the amounts of sesamin and episesamin were determined from the ratio between the peak area of sesamin or episesamin and the peak area of the internal standard YUDESMIN (Funakoshi Corporation). The conditions for LC-MS/MS analysis are shown below.
(HPLC)
Column: Develosil C30-UG-5 (5 µm, 2.0 Φ x 50 mm; product of NOMURA CHEMICAL CO., LTD.)
Mobile phase: A, distilled water; B, methanol; D, 100 mM ammonium acetate in water
Flow rate: 0.25 mL/min
Gradient: Linear gradient consisting of 2 minutes with 55% fluid B and 10% fluid D, followed by 3 minutes with fluid B changing from 55% to 60% but fluid D remaining at 10%, then 2 minutes with fluid B changing from 60% to 85% but fluid D remaining at 10%.
(MS/MS)
Measurement mode: Monitoring of selective reaction
Detection: sesamin (about 5.0 min of retention time);
precursor ion, m/z = 372 ([M+NH₄]+), generated ion, m/z = 233.
: episesamin (about 5.4 min of retention time);
precursor ion, m/z = 372 ([M+NH₄]+), generated ion, m/z = 233.
: YUDESMIN (about 2.6 min of retention time);
precursor ion, m/z = 369 ([M+NH₄]+), generated ion, m/z = 298.
Ionizing method: ESI method
FIG. 1 shows the time course of the total sum of sesamin and episesamin levels (sesamin + episesamin level) in blood. The maximum value (Cmax) of sesamin + episesamin level in blood was 20 ng/mL in the comparative sample ingesting group but 35 ng/mL in the test sample ingesting group. The time (Tmax) to the maximum value (Cmax) was 5 hours in each of the test sample ingesting group and the comparative sample ingesting group. Furthermore, the amount of bodily absorption (AUC) was determined from FIG. 1 and it was found to be about 1.6 times higher in the test sample ingesting group than in the comparative sample ingesting group.

As shown in the foregoing, the group that ingested the test sample (the composition of the present invention) featured enhanced sesamin absorption, thus suggesting their capability for efficient ingestion of sesamin.

### [Reference Example: Absorption Test-1>

### Samples

One gram of sesamin (product of TAKEMOTO OIL & FAT Co., Ltd.; sesamin/episesamin = 51.1:48.2) was suspended in 50 g of olive oil that had been heated to 80 °C and the suspension was agitated for 20 minutes until the sesamin dissolved uniformly. The resulting solution was cooled to about 70 °C and poured under agitation into an aqueous solution prepared by mixing and dissolving 25 g of enzyme-decomposed lecithin (SUN LECITHIN VA-1; product of Taiyo Kagaku Co., Ltd.; 33.3% active ingredient; obtained from soybean) in 1000 mL of water that had been heated to 70 °C, and the mixture was emulsified at 6000 rpm for 15 minutes with Distromix (product of ATEC JAPAN Co., Ltd.) The emulsified liquid was held at 50-60 °C and processed with a high-speed agitating emulsifier (CLEAR MIX W-Motion, product of M Technique) for 40 minutes with the rotor part running at 20000 rpm and the screen part at 12500 rpm, whereby a sesamin-containing, water-soluble emulsified composition (sesamin-containing, oil-in-water emulsion) was obtained (sample 1). The average particle size of the obtained sesamin-containing, oil-in-water emulsion was measured with the dynamic light scattering nanoparticle size analyzer Model LB-550 of HORIBA, Ltd. and the result was 97.8 nm.

For comparison, 50 mg of sesamin (product of TAKEMOTO OIL & FAT Co., Ltd.; sesamin/episesamin = 51.1:48.2) was suspended in 50 mL of olive oil that had been heated to 80°C and the suspension was agitated for 20 minutes until the sesamin dissolved uniformly (comparative sample).

### Sesamin's bodily absorption test

SD (IGS) male rats (9-week old) were purchased from CHARLES RIVER LABORATORIES, JAPAN, INC. and acclimatized in the test environment for a week; the animals that were shown to have grown normally were subjected to the test. The rats that were fasted overnight were divided into two groups, each consisting of 4 animals, and using a stomach tube, they were perorally administered with the sesamin-containing, oil-in-water emulsion as sample 1 or the olive-dissolved fat or oil of sesamin as the comparative sample at a dose of 10 mg/10 mL/kg. At 1, 3, 5, 7, 9 and 25 hours after the start of administration, blood was withdrawn from the tail vein of each animal, collected into a heparinized blood collecting tube, and centrifuged (8000 rpm, 10 min) to obtain plasma samples. After adding an internal standard, the solid phase was extracted with Oasis HLB and the liquid extract was concentrated under vacuum; the concentrate was suspended in methanol, passed through a filter and subjected to LC-MS/MS to quantitate sesamin and its isomer, episesamin. According to the usual method, the amounts of sesamin and episesamin were determined from the ratio between the peak area of sesamin or episesamin and the peak area of the internal standard YUDESMIN (Funakoshi Corporation). The conditions for LC-MS/MS analysis are shown below.

(HPLC)
Column: Develosil C30-UG-5 (5 µm, 2.0 Φ x 50 mm; product of
NOMURA CHEMICAL CO., LTD.)
Mobile phase: A, distilled water; B, methanol; D, 100 mM
ammonium acetate in water
Flow rate: 0.25 mL/min
Gradient: Linear gradient consisting of 2 minutes with 55% fluid B and 10% fluid D, followed by 3 minutes with fluid B changing from 55% to 60% but fluid D remaining at 10%, then 2 minutes with fluid B changing from 60% to 85% but fluid D remaining at 10%.
(MS/MS)
Measurement mode: Monitoring of selective reaction
Detection: sesamin (about 5.2 min of retention time);
precursor ion, m/z = 372 ([M+NH₄]+), generated ion, m/z = 233.
:episesamin (about 5.6 min of retention time);
precursor ion, m/z = 372 ([M+NH₄]+), generated ion, m/z = 233.
: YUDESMIN (about 2.8 min of retention time);
precursor ion, m/z = 404 ([M+NH₄]+), generated ion, m/z = 249.
Ionizing method: ESI method
FIG. 3 shows the time course of the total sum of sesamin and episesamin levels (sesamin + episesamin level) in blood. The maximum value (Cmax) of sesamin + episesamin level in blood was 48 ng/mL in the sample 1 ingesting group but 20 ng/mL in the comparative sample ingesting group. The time (Tmax) to the maximum value (Cmax) was about one hour in the sample 1 ingesting group but about nine hours in the comparative sample ingesting group. Furthermore, the amount of bodily absorption (AUC) was determined from FIG. 3 and it was found that there was no difference in the amount of absorption.

### [EXAMPLE 3] Formulations

### (Preparation 1) Capsules

| | |
|---|---|
| Gelatin | 60.0% |
| Glycerin | 30.0% |
| Methyl paraoxybenzoate | 0.15% |
| Propyl paraoxybenzoate | 0.51% |
| Water | q.s. |

Thirty milliliters of a 1% sesamin containing oil-soluble emulsified composition (O/W/O emulsion) was obtained in accordance with the method of sample preparation described in Example 1, except that 1.362 g of sesamin (product of TAKEMOTO OIL & FAT Co., Ltd.; sesamin/episesamin = 51.1:48.2) was suspended in 100 mL of olive oil that had been heated to 80°C. The composition was then filled into soft capsules of gelatin shell consisting of the above-mentioned ingredients by a conventional method to prepare soft capsules each weighing 250 mg. The thus prepared soft capsules each contain 2.5 mg of sesamin.

### (Preparation 2) Mayonnaise

| | |
|---|---|
| Egg yolks | 10 |
| Sodium chloride | 16 g |
| Mustard | 25 g |
| Pepper | 3 g |
| Vinegar | 150 cc |

After fully agitating the above-mentioned ingredients, 1000 cc of a 0.1% sesamin containing oil-soluble emulsified composition (O/W/O emulsion) and 500 cc of an edible vegetable oil were added dropwise to a water phase under agitation, followed by homogenization with a stirrer to obtain sesamin-containing mayonnaise.

### (Preparation 3) Butter

### 1% sesamin containing oil-soluble

| | |
|---|---|
| emulsified composition (O/W/O emulsion) | 5 g |
| Butterfat | 95 g |
| Tocopherol acetate | 1.2 g |

To 95 g of butterfat separated from buttermilk by the churning step in the process of butter production, 5 g of the 1% sesamin containing oil-soluble emulsified composition (O/W/O emulsion) and 1.2 g of the tocopherol acetate were added and the mixture was worked until a homogeneous texture was obtained, yielding butter containing the composition of the present invention.

### (Preparation 4) Margarine

| | |
|---|---|
| Vegetable oil or fat (hardened soybean oil mixed with cottonseed oil) | 72 wt% |
| 1% sesamin containing oil-soluble emulsified composition (O/W/O emulsion) | 10 wt% |
| Glycerin fatty acid ester | 0.2 wt% |
| Skim milk powder | 0.5 wt% |
| Water | 17 wt% |
| Sodium chloride | 0.8 wt% |

To the molten vegetable oil or fat, the 1% sesamin containing oil-soluble emulsified composition (O/W/O emulsion) and the glycerin fatty acid ester were added and the mixture was stirred; thereafter, the skim milk powder and the sodium chloride as dissolved in water were gradually added and the ingredients were stirred under heating at 50-60°C to form a mixture. The mixture was quenched to plasticize, yielding margarine containing the composition of the present invention.

## Claims

1. An O/W/O emulsion composition having at least one of lignan-class compounds dissolved in an internal oil phase.

2. The O/W/O emulsion composition according to claim 1, wherein the lignan-class compound is sesamin and/or episesamin.

3. The O/W/O emulsion composition according to claim 1 or 2, wherein at least one of the internal oil phase, water phase and/or external oil phase contains a surfactant in an amount effective for emulsification.

4. The O/W/O emulsion composition according to any one of claims 1-3, wherein the internal oil phase has an average particle size of 1000 nm or smaller (preferably 500 nm or smaller, and more preferably 300 nm or smaller).

5. The O/W/O emulsion composition according to any one of claims 1-4 in the form of a food composition or an oral pharmaceutical composition.

6. An O/W/O emulsion composition according to claim 1 comprising an internal oil phase which is emulsified in a water phase to make an O/W emulsion composition which in turn is emulsified in an external oil phase, the internal oil phase containing at least one of lignan-class compounds.

7. An O/W/O emulsion composition according to claim 1 having at least one of lignan-class compounds dissolved in an internal oil phase in a clinically effective amount, said composition optionally containing a surfactant in an amount effective for emulsification, which composition, when administered perorally, enables at least one of lignan-class compounds to be absorbed with a greater area under the blood concentration-time curve (AUC) than when the same quantity of that lignan-class compound is dissolved in the same oil or fat as the internal oil phase and administered perorally under the same conditions.

8. A process for producing a lignan-class compound containing composition comprising the following steps:
1) dissolving at least one of lignan-class compounds in oil or fat to prepare a lignan-class compound dissolving liquid;
2) emulsifying the lignan-class compound dissolving liquid in a water phase to form an O/W emulsion; and
3) further emulsifying the O/W emulsion in an oil phase to prepare an O/W/O emulsion.

9. The process according to claim 8, wherein the lignan-class compound is sesamin and/or episesamin.

10. The O/W/O emulsion composition according to claim 1 for use in improving the amount of bodily absorption of lignan-class compounds.

11. The O/W/O emulsion composition according to claim 10, wherein the lignan-class compound is sesamin and/or episesamin.

## Patentansprüche

1. ONV/O-Emulsionszusammensetzung mit mindestens einer Verbindung aus der Klasse der Lignane, welche in einer inneren Ölphase gelöst ist.

2. O/W/O-Emulsionszusammensetzung nach Anspruch 1, wobei die Verbindung aus der Klasse der Lignane Sesamin und/oder Episesamin ist.

3. O/W/O-Emulsionszusammensetzung nach Anspruch 1 oder 2, wobei mindestens eine von der inneren Ölphase, der wässrigen Phase und/oder der äußeren Ölphase einen grenzflächenaktiven Stoff in einer zum Emulgieren wirksamen Menge enthält.

4. O/W/O-Emulsionszusammensetzung nach einem der Ansprüche 1-3, wobei die innere Ölphase eine durchschnittliche Partikelgröße von 1000 nm oder kleiner (vorzugsweise 500 nm oder kleiner, und stärker bevorzugt 300 nm oder kleiner) hat.

5. O/W/O-Emulsionszusammensetzung nach einem der Ansprüche 1-4 in Form einer Nahrungsmittelzusammensetzung oder eines oralen Arzneimittels.

6. O/W/O-Emulsionszusammensetzung nach Anspruch 1, umfassend eine innere Ölphase, die in einer wässrigen Phase emulgiert ist, um eine O/W-Emulsionszusammensetzung zu ergeben, die wiederum in einer äußeren Ölphase emulgiert ist, wobei die innere Ölphase mindestens eine Verbindung aus der Klasse der Lignane enthält.

7. O/W/O-Emulsionszusammensetzung nach Anspruch 1 mit mindestens einer Verbindung aus der Klasse der Lignane, die in einer inneren Ölphase gelöst ist, in einer klinisch wirksamen Menge, wobei die Zusammensetzung gegebenenfalls einen grenzflächenaktiven Stoff in einer zum Emulgieren wirksamen Menge enthält, wobei die Zusammensetzung, wenn sie peroral verabreicht wird, ermöglicht, dass mindestens eine Verbindung aus der Klasse der Lignane mit einer größeren Fläche unter der Blutspiegel-Zeit-Kurve (AUC) absorbiert wird, als wenn die gleiche Menge dieser Verbindung aus der Klasse der Lignane im gleichen Öl oder Fett als die innere Ölphase gelöst ist und peroral unter den gleichen Bedingungen verabreicht wird.

8. Verfahren zur Herstellung einer eine Verbindung aus der Klasse der Lignane enthaltenden Zusammensetzung, umfassend die folgenden Schritte:
1) Lösen von mindestens einer Verbindung aus der Klasse der Lignane in Öl oder Fett, um eine Flüssigkeit herzustellen, welche die Verbindung aus der Klasse der Lignane löst;
2) Emulgieren der Flüssigkeit, welche die Verbindung aus der Klasse der Lignane löst, in einer wässrigen Phase, um eine O/W-Emulsion zu bilden; und
3) weiterhin Emulgieren der O/W-Emulsion in einer Ölphase, um eine O/W/O-Emulsion herzustellen.

9. Verfahren nach Anspruch 8, wobei die Verbindung aus der Klasse der Lignane Sesamin und/oder Episesamin ist.

10. O/W/O-Emulsionszusammensetzung nach Anspruch 1 zur Verwendung für die Verbesserung der durch den Körper aufgenommenen Absorptionsmenge von Verbindungen aus der Klasse der Lignane.

11. O/W/O-Emulsionszusammensetzung nach Anspruch 10, wobei die Verbindung aus der Klasse der Lignane Sesamin und/oder Episesamin ist.

## Revendications

1. Composition d'émulsion H/E/H comprenant au moins un des composés de la classe des lignanes dissous dans une phase huileuse interne.

2. Composition d'émulsion H/E/H selon la revendication 1, dans laquelle le composé de la classe des lignanes est la sésamine et/ou l'épisésamine.

3. Composition d'émulsion H/E/H selon la revendication 1 ou 2, dans laquelle au moins l'une de la phase huileuse interne, la phase aqueuse et/ou la phase huileuse externe contient un tensioactif en une quantité efficace pour une émulsification.

4. Composition d'émulsion H/E/H selon l'une quelconque des revendications 1 à 3, dans laquelle la phase huileuse interne possède une taille de particule moyenne de 1 000 nm ou moins (de préférence 500 nm ou moins et, de manière davantage préférée, 300 nm ou moins).

5. Composition d'émulsion H/E/H selon l'une quelconque des revendications 1 à 4, sous la forme d'une composition alimentaire ou d'une composition pharmaceutique orale.

6. Composition d'émulsion H/E/H selon la revendication 1, comprenant une phase huileuse interne qui est émulsifiée dans une phase aqueuse pour obtenir une composition d'émulsion H/E qui, à son tour, est émulsifiée dans une phase huileuse externe, la phase huileuse interne contenant au moins un des composés de la classe des lignanes.

7. Composition d'émulsion H/E/H selon la revendication 1, comprenant au moins un des composés de la classe des lignanes dissous dans une phase huileuse interne en une quantité cliniquement efficace, ladite composition contenant facultativement un tensioactif en une quantité efficace pour une émulsification, où la composition, lorsqu'elle est administrée par voie orale, permet à au moins un des composés de la classe des lignanes d'être absorbé avec une plus grande aire sous la courbe concentration sanguine-temps (ASC) par rapport à la situation où la même quantité de ce composé de la classe des lignanes est dissoute dans la même huile ou graisse que la phase huileuse interne puis est administrée par voie orale dans les mêmes conditions.

8. Procédé destiné à produire une composition contenant un composé de la classe des lignanes, comprenant les étapes suivantes :
1) dissoudre au moins un des composés de la classe des lignanes dans une huile ou une graisse afin de préparer un liquide de dissolution d'un composé de la classe des lignanes ;
2) émulsifier le liquide de dissolution d'un composé de la classe des lignanes dans une phase aqueuse afin de former une émulsion H/E ; et
3) émulsifier en outre l'émulsion H/E dans une phase huileuse afin de préparer une émulsion H/E/H.

9. Procédé selon la revendication 8, dans lequel le composé de la classe des lignanes est la sésamine et/ou l'épisésamine.

10. Composition d'émulsion H/E/H selon la revendication 1, destinée à être utilisée pour améliorer la quantité d'absorption corporelle de composés de la classe des lignanes.

11. Composition d'émulsion H/E/H selon la revendication 10, dans laquelle le composé de la classe des lignanes est la sésamine et/ou l'épisésamine.
